# EUROPEAN PATENT APPLICATION

(11) **EP 1 889 562 A1**
(43) Date of publication of application: **20.02.2008**
(21) Application number: 06746654.0
(22) Date of filing: 19.05.2006
(51) Int. Cl.: A61B 1/00, A61B 5/07

(54) **MEDICAL CAPSULE ENDOSCOPE**

(30) Priority: 08.06.2005 JP 2005167993
(71) Applicant: Konica Minolta Medical & Graphic, Inc., Tokyo 163-0512 (JP)
(72) Inventor: HABU, Takeshi Konica Minolta Medical & Graphic,Inc, Tokyo 1630512 (JP); SAKUMA, H. Konica Minolta Medical & Graphic, Inc., Tokyo 1630512 (JP); TAKEYAMA, T. Konica Minolta Medical & Graphic, Inc, Tokyo 1630512 (JP); GOTO, Narito Konica Minolta Medical & Graphic, Inc, Tokyo 1630512 (JP); UMEKI, Mamoru Konica Minolta Medical & Graphic,Inc, Tokyo 1630512 (JP)
(74) Representative: Alton, Andrew
(86) International application number: PCT/JP2006/309998
(87) International publication number: WO 2006/132066

(57) **Abstract**

An objective is to provide a medical capsule endoscope exhibiting excellent image quality of photographed images such as sharpness, and also exhibiting excellent washability capable of quickly washing and sterilizing after the removal from a human body. Also disclosed is a medical capsule endoscope possessing a surface having a contact angle of not less than 60° and not more than 150° with respect to water.

## Description

### TECHNICAL FIELD

The present invention relates to a capsule endoscope whose surface is hydrophobicity-treated, and specifically to a medical capsule endoscope in the form of tablet capsule integrated with an objective lens, an illuminating source section, a solid-state image sensor and so forth

### BACKGROUND

Generally, a conventional endoscopic apparatus comprises a tubular insertion section equipped with an image sensor or such at the end, an operation section connected to this insertion section, an image processor connected thereto, a display apparatus and so forth, and practically utilized has been the endoscopic apparatus to inspect and observe desired portions in the inside of body cavity by inserting the insertion section into the inside of body cavity of an object to sense images. In such the endoscopic apparatus, sufficiently skilled doctors and engineers should have operated it because of a large size and length of the insertion section inserted in the inside of body cavity, and its complicated shape to conduct inspection and observation. These apparatuses also cause pain to the object during insertion to the inside of body cavity.

In view of such the situation, for example, recently developed has been an ultrasmall endoscope, which is a so-called capsule endoscope, fitted with a solid-state image sensor having a photographic optics system in the inside of a tablet-capsule-shaped enclosure and so forth. The capsule endoscope swallowed by the object is inserted into the inside of body cavity. By this, organs such as a small intestine and so forth have easily been observed and inspected employing a wireless communication device by which images of an affected area are taken, the image data are transmitted from the inside of a body, and the signal is received outside the body (refer to Patent Document 1, for example).

However, though it is mentioned in techniques with regard to patents which have been disclosed so far that the outermost surface of the capsule endoscope exhibits high hydrophilicity, no surface condition related technique on which is intensively focused has been known so far. The capsule endoscope conducts photographing in the inside of a body cavity, inspection and operation with a very small machine, but it is removed outside the body cavity, that is, outside a human body after use. When the inspection or operation has been conducted, the capsule endoscope has to be collected. In this case, washing and a sterilization operation are desired to be conducted. There is a problem of how quickly and effectively the surface is washed and sterilized to inspect a biopsied body stored in the capsule and operation parts. There was a balancing problem between being able to photograph the inside precisely and washing the outer surface after collection. When the surface is arranged to be water-repellent in order to improve washability, it is easy to be contaminated with secretion such as bile during movement in the inside of body cavity, whereby interference with photographing was caused because of mist of the lens. A digital camera was able to avoid a problem of blurring images due to hand movement by introducing a hand movement correcting mechanism, but there was a problem concerning controlling of image shake caused by a moving living body, since it was difficult to build a complex mechanism into the capsule endoscope.
(Patent Document 1) Japanese Patent O.P.I. Publication No. 2001-091860

### DISCLOSURE OF THE INVENTION

### PROBLEMS TO BE SOLVED BY THE INVENTION

The present invention was made on the basis of the above-described situation. It is an object of the present invention to provide a medical capsule endoscope exhibiting excellent image quality of photographed images such as sharpness, and also exhibiting excellent washability capable of quickly washing and sterilizing after the removal from a human body.

### MEANS TO SOLVE THE PROBLEMS

The above-described object can be accomplished by the following structures.
(Structure 1) A medical capsule endoscope comprising a surface having a contact angle of not less than 60° and not more than 150° with respect to water.
(Structure 2) The medical capsule endoscope of Structure 1, wherein the surface comprises a surface of a lens or an illumination body provided in the medical capsule endoscope.
(Structure 3) The medical capsule endoscope of Structure 1 or 2, wherein a chemically bonded fluorine atom is present on the surface.
(Structure 4) The medical capsule endoscope of any one of Structures 1 - 3, wherein the surface is treated via a microwave plasma treatment, an electron beam treatment or an X-ray treatment.

### EFFECTS OF THE INVENTION

In the present invention, produced can be a medical capsule endoscope exhibiting excellent image quality of photographed images such as sharpness, and also exhibiting excellent washability capable of quickly washing and sterilizing after the removal from a human body.

### BRIEF DESCRIPTION OF THE DRAWINGS

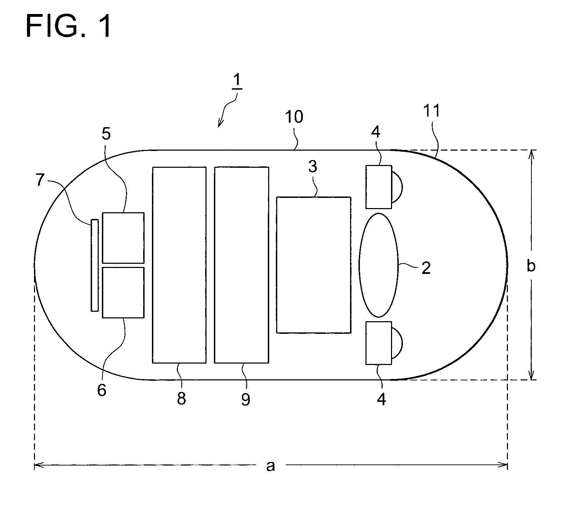

Fig. 1 is an illustrative diagram showing an example of a structure of a medical capsule endoscope in the present invention.

### EXPLANATION OF NUMERALS

1 Capsule endoscope
2 Objective lens
3 Image sensor
4 Light emission module
5 Transmitting device
6 Receiving device
7 Antenna
8 Electric storage device
9 Space area
10 Capsule endoscope enclosure
11 Surface member of an observation section and an illumination section

### DESCRIPTION OF THE PREFERRED EMBODIMENTS

Next, an outline of a medical capsule endoscope of the present invention will be described referring to a figure.

Fig. 1 is an illustrative diagram showing an example of a structure of a medical capsule endoscope in the present invention. As shown in Fig. 1, medical capsule endoscope 1 (hereinafter, also referred to simply as a capsule endoscope) of the present invention is a so-called capsule endoscope capable of wireless transmitting and receiving images, and CCD or CMOS is employed for a image sensor constituting observation optical system (image sensor) 3 as an observing function device. A white, green or red LED (hereinafter, also referred to collectively as LED) is utilized for an illumination device constituting illumination optical system (light emission module) 4. Wireless transmitting and receiving antenna 7 is placed with receiving device (receiving means) 5, transmitting device (transmitting means) 6, and electric storage device (electric storage means) 8 provided on the opposite side of the illumination system. As space area 9, provided is a room of storing a medical solution to be fed to the affected area, or provided is, in some situations, a room of storing the specimen after collecting a test-specimen in the affected area. Electric storage device 8 may be a transformer converting into an electric source to produce light emission of LED for illumination by an ultrasonic wave, magnetic field or electric field transmitted from the outside. The capsule endoscope is in the form of a small chicken egg so as to move smoothly in the upper and lower digestive organs, and the rough elliptical shape is preferable as a top view as well as a side view, but the spherical shape or rugby ball shape may be allowed to be used. An a/b ratio of not less than 1.0 and not more than 2.3 is preferable, but an a/b ratio of not less than 1.0 and not more than 2.0 is more preferable, provided that the a/b ratio is defined as an aspect ratio, representing the outer diameter of the short axis and the outer diameter of the long axis as b and a, respectively.

Concerning a lens to focus an observed image onto CCD or CMOS as an image sensor, a single lens or a plurality of aspheric objective lenses, for example, is/are provided. In the case of employing these aspheric lenses, since a number of lenses can be reduced, the total length of a capsule section can be shortened, whereby a swallowable property can be improved. Forgoing capsule endoscope 1 includes an endoscope observation system fitted with an output signal processing system to drive image sensor 3, an electric installation device to supply electric power to illumination device 4, and a liquid crystal display element in combination to sharply display endoscope images photographed by foregoing image sensor 3 on a liquid crystal monitor placed outside a body.

For example, endoscope images in photographed recording are recorded onto a recording medium such as a compact flash memory, and image-processed to detect a diseased portion, whereby early detection becomes possible. The electric installation device is driven by a commercial electric source, an electric source for household use, a battery or such.

Enclosure 10 of the capsule endoscope, which is formed from a chicken egg shaped, an elliptically shaped, a roundly shaped, or a rugby ball shaped protective member is provided in the form of covering the exterior of an observing function device (an illumination optical system and an observation optical system), space area 9 and a transmission/reception system constituting above-described capsule endoscope 1. Enclosure 10 of this capsule endoscope is preferably made of a resin material, and usable examples thereof include a methyl polymethacrylate resin, a polyethylene resin, a polypropylene resin, a polyethylene terephthalate resin, a nylon resin and so forth. Further, surface member 11 of an observation section and an illumination section is provided in order to protect the observing function device (an illumination optical system and an observation optical system) specifically in enclosure 10 of this capsule endoscope. A material constituting enclosure 10 of this capsule endoscope and a material constituting surface member 11 of the observation section and the illumination section may be identical or different, but a resin material exhibiting high transparency (high transmittance) is desired to be used for at least surface member 11 of the observation section and the illumination section.

It is preferable in a capsule endoscope of the present invention that a hydrophobic medium is coated or is provided via a surface treatment on the surface formed from a resin material constituting at least one of enclosure 10 of the capsule enclosure and surface member 11 of the observation section and the illumination section in the capsule of capsule endoscope 1, since formed is the surface having a contact angle of not less than 60° and not more than 150° with respect to water specified in the present invention. As to this hydrophobic treatment, surface member 11 of the observation section and the illumination section as a lens and an illumination body preferably takes priority over. The entire outermost surface of enclosure 10 of the capsule endoscope, and surface member 11 of the observation section and the illumination section is more preferably subjected to a hydrophobic treatment. In this way, when swallowing capsule endoscope 1, it passes through a digestive tract without contaminating a lens and a illumination window of foregoing capsule endoscope 1, and can be smoothly moved with no damage, since the medium repels water such as secretion and bile in the inside of digestive organs.

The hydrophobic treatment for the capsule endoscope surface can be conducted by various methods. Examples thereof include a method of coating a hydrophobizing agent on the capsule endoscope surface, and a method of bonding a hydrophobizing agent and a functional group in a resin on the surface, and bonding can be performed in chemical reaction via addition and condensation. Further, examples of the method of treating via the chemical reaction on the surface of an object, utilizing radiation and so forth include a microwave plasma treatment method and treating methods employing electron beam, X-ray and so forth.

The microwave plasma treatment method, for example, can draw upon an apparatus described in Japanese Patent O.P.I. Publication No. 2005-002355. The microwave preferably has a frequency of 2.45 GHz, and preferably has an output power of 100 - 2000 W, depending on the number to be treated. The level of reduced pressure during treatment may be at a level where a glow discharge is generated when microwaves are introduced after introducing gas for treatment. Specifically, a reduced pressure of 0.1 - 100 Pa is used, and a reduced pressure of 1 - 10 Pa is preferably used. The gas for treatment is supplied at a flow rate of 0.01 - 10 ml/min per a capsule endoscope vessel under the standard condition, and preferably supplied at a flow rate of 0.1 - 1 ml/min per a capsule endoscope vessel under the standard condition. In the case of conducting a hydrophilic treatment with a plurality of gases for treatment, a gas for treatment may be excessively supplied. For example, in the case of forming a silicon oxide film, it is preferred that oxygen gas is excessively supplied with respect to silicon source gas, and the oxygen gas is supplied at a flow rate of 0.1 - 100 ml/min per a capsule endoscope vessel under the standard condition, and preferably supplied at a flow rate of 1 - 10 ml/min per a capsule endoscope vessel under the standard condition.

Hydrogen fluoride gas can be employed in order to conduct a fluorination treatment on the capsule endoscope surface to chemically bond a fluorine atom. In the xase of an electron beam treatment, a low molecular compound having a fluorine atom together with easy gasfication is preferable as a water repellent. Specific examples thereof include fluoride gas designated as F-1 (others designated as the following numerals) such as hydrogen fluoride (F-2), nitrogen trifluoride (F-3), boron trifluoride (F-4), sulfur tetrafluoride (F-5) or silicon tetrafluoride (F-6); and chlorofluorocarbon such as dichlorodifluoromethane (F-7), dichloromonofluoromethane (F-8), monochlorodifluoromethane (F-9), trifluoromethane (F-10), difluoromethane (F-11), tetrachlorodifluoroethane (F-11) or hexafluoroethane (F-12).

As electron beam employed for an electron beam treatment preferably used in a water repellency processing treatment of the present invention, electron beam generated from an electron-ray tube is preferably used. There are two types in the electron-ray tube such as a field emission type and a thermoelectric emission type. In any of the types, an electron beam source equipped with a cathode and an anode is installed in a chamber, and an irradiated material is placed in the chamber. As a field emission type electron-ray tube, known is one described in Fig. 1 of Published Japanese translation of PCT International Publication No. 11-505670. In the case of the field emission type electron-ray tube, the inside of a chamber in which an irradiated material is placed needs to be highly vacuated.

On the other hand, as a thermoelectronic emission type electron-ray tube described in Published Japanese translation of PCT International Publication No. 10-512092, for example, a vacuum tube type electron-ray tube having a window has become commercially available. The above-described electron-ray tube is designed to emit thermoelectrons from the above-described window by accelerating thermoelectrons emitted from a thermoelectron emission section with an electron beam acceleration section after installing the thermoelectron emission section and the electron beam acceleration section in a vacuum chamber having a window letting electrons pass through. By using such the electron-ray tube, electron beam can be taken out in atmosphere at normal pressure from a window of the electron-ray tube. Accordingly, a single structure of an electron beam irradiation treatment apparatus, together with easy handling can be realized since no atmosphere in which an irradiated material is placed needs to be depressurized, and a vacuum pump and a vacuum chamber employed for reduced pressure are not required to be used.

As for an X-ray treatment applicable in the present invention, a low energy X-ray is produced from high energy electrons obtained by generating plasma in a band width divided by a mirror magnetic field as shown in Japanese Patent O.P.I. Publication No. 07-080050, and an irradiated material may be exposed to the low energy X-ray via an X-ray transmitting barrier. In this case, a water repellent employed during the above-described plasma treatment can be used.

When conducting a water repellent process, it is a feature that the above-described treatment is carried out so as to give the surface a contact angle of not less than 60° and not more than 150°. In the case of a contact angle of less than 60°, there is a contamination problem caused by bile, and in the case of a contact angle exceeding 150°, no image exhibiting high sharpness can be obtained because of no smooth movement of the capsule endoscope in a body cavity. In order to measure the contact angle on the surface of the capsule endoscope, a boundary between solids, as well as between vapor and liquid, is automatically determined from liquid droplet images stored by a CCD camera employing OCA20 manufactured by IPROS Corporation to measure the contact angle via curve fitting. It is preferable in the present invention that the surface is fluorination-treated to chemically bond a fluorine atom and the capsule surface. Incidentally, presence of a fluorine atom on the surface can be confirmed by a surface analysis method via electron spectroscopy or such.

### EXAMPLE

### «Preparation of capsule endoscope»

### [Preparation of sample 101]

A capsule endoscope composed of a structure described in Fig. 1 was prepared. Enclosure 10 (area: 70%) of this capsule endoscope and surface member 11 (area: 30%) of an observation section and an illumination section were formed from a methyl polymethacrylate resin.

Next, the entire surface (treated area: 100%) of enclosure 10 and surface member 11 was water-repellency-treated under the conditions of a reduced pressure of 5 Pa and a flow rate of 1 ml/sec with fluorine gas for 0.1 sec., employing a plasma treatment apparatus to generate plasma by using microwaves having a frequency 2.45 GHz to prepare sample 101.

### [Preparation of samples 102 - 104, 107 and 108]

Samples 102 - 104, 107 and 108 were prepared similarly to the above-described preparation of sample 101, except that the plasma-treating time with fluorine gas was replaced by 0.2 sec, 0.3 sec, 0.4 sec, 0.5 sec and 0.6 sec, respectively.

### [Preparation of sample 105]

Sample 105 was prepared similarly to the above-described preparation of sample 104, except that as the surface water repellency treatment method, the plasma treatment method was replaced by an electron beam treatment method employing a Min-EB radiation system (manufactured by Ushio Inc.) operated at 50 keV with exposure at 2 Mgy.

### [Preparation of sample 106]

Sample 106 was prepared similarly to the above-described preparation of sample 104, except that as the surface water repellency treatment method, the plasma treatment method was replaced by an X-ray treatment method employing DINAMITRON (manufactured by RDI) with exposure at 2 Mgy.

### [Preparation of samples 109 - 111]

Samples 109 - 111 were prepared similarly to the above-described preparation of sample 101, except that as a surface treatment material used during the plasma treatment, the fluorine gas was replaced by silane gas, methyl fluoride solane gas and fluorinated propylene gas, respectively, and the surface water repellency treatment time was further replaced by 1.0 sec.

### [Preparation of samples 112 - 114]

Samples 112 - 114 were prepared similarly to the above-described preparation of sample 101, except that the surface water repellency treatment time was replaced by 1.0 sec., and the treated area was further replaced by 70%, 40%, and 20%, respectively, while occluding enclosure 10 (area: 70%) of a capsule endoscope and surface member 11 (area: 30%) of an observation section and an illumination section with an aluminum foil during plasma treatment. In addition, The starting point for occluding was set to the most-distant position from the center of the lens.

### [Preparation of sample 115]

A capsule endoscope composed of enclosure 10 (area: 70%) and surface member 11 (area: 30%) of an observation section and an illumination section in Fig. 1 which were made of a methyl polymethacrylate resin was immersed in an aqueous 5% by weight fluorinated acid solution at 23 °C for 20 seconds so as to give a treated area of 70% (the entire area of surface member 11 and 4/7 of enclosure 10), and subsequently dried to prepare sample 115. This method is called a wet treatment method.

### [Preparation of sample 116]

A capsule endoscope composed of enclosure 10 (area: 70%) and surface member 11 (area: 30%) of an observation section and an illumination section in Fig. 1 which were made of a methyl polymethacrylate resin was immersed in an aqueous 5% by weight fluorinated alcohol solution at 23 °C for 20 seconds so as to give a treated area of 70% (the entire area of surface member 11 and 4/7 of enclosure 10), and subsequently dried to prepare sample 116. This method is called a wet treatment method.

### «Evaluation of capsule endoscope»

### [Measurement of contact angle]

The contact angle at a portion where a surface water repellency treatment was conducted for each sample was measured. An boundary between solids, as well as between vapor and liquid, is automatically determined from liquid droplet images stored by a CCD camera employing OCA20 manufactured by IPROS Corporation as a contact angle measuring device to measure a contact angle with respect to water via curve fitting.

### [Evaluation of washability]

Each capsule endoscope used for artificial digestive organs was passed through the digestive organs, and subsequently washed employing a 50% ethanol solution as a sterilization cleaning solution for each capsule endoscope, and then a cleanliness level of the surface of each capsule endoscope was measured by a checking meter WPTF-20 manufactured by Shiro Co., Ltd. The smaller the measured ATP value, the higher the cleanliness level is.

### [Evaluation of image]

Artificial digestive organs were used for photographing in the inside of digestive organs, and sharpness of transmitted photographed images was visually observed to evaluate images in accordance with the following criteria. In addition, rank 2.5 means a ranking value intermediate between rank 2 and rank 3, and indicates image quality outside the range practically accepted.
5: The transmitted image exhibits extremely high sharpness.
4: The transmitted image exhibits high sharpness.
3: The transmitted image exhibits slightly degraded sharpness, but is practically accepted.
2: The transmitted image exhibits degraded sharpness.
1: The transmitted image exhibits clearly degraded sharpness, and is practically undurable.

**Table 1**

| Sample No. | Experimental condition | | | Evaluated results | | | | *4 |
|---|---|---|---|---|---|---|---|---|
| | Hydrophobic process | Treating material | *1 | Contact angle (°) | Treated area (%) | *2 | *3 | |
| 101 | Plasma treatment | Fluorine gas | 0.1 | 40 | 100 | 2.5 | 150000 | Comp. |
| 102 | Plasma treatment | Fluorine gas | 0.2 | 60 | 100 | 3 | 2200 | Inv. |
| 103 | Plasma treatment | Fluorine gas | 0.3 | 80 | 100 | 4 | 532 | Inv. |
| 104 | Plasma treatment | Fluorine gas | 0.4 | 120 | 100 | 5 | 340 | Inv. |
| 105 | Electron Beam treatment | Fluorine gas | 0.4 | 120 | 100 | 5 | 340 | Inv. |
| 106 | X-ray treatment | Fluorine gas | 0.4 | 120 | 100 | 5 | 340 | Inv. |
| 107 | Plasma treatment | Fluorine gas | 0.5 | 150 | 100 | 4 | 230 | Inv. |
| 108 | Plasma treatment | Fluorine gas | 0.6 | 160 | 100 | 2.5 | 120 | Comp. |
| 109 | Plasma treatment | Silane gas | 1.0 | 120 | 100 | 5 | 120 | Inv. |
| 110 | Plasma treatment | Methyl fluoride silane | 1.0 | 120 | 100 | 5 | 120 | Inv. |
| 111 | Plasma treatment | Fluorinated propylene | 1.0 | 120 | 100 | 5 | 120 | Inv. |
| 112 | Plasma treatment | Fluorine gas | 1.0 | 120 | 70 | 5 | 120 | Inv. |
| 113 | Plasma treatment | Fluorine gas | 1.0 | 120 | 40 | 4 | 120 | Inv. |
| 114 | Plasma treatment | Fluorine gas | 1.0 | 120 | 20 | 2.5 | 120 | Comp. |
| 115 | Wet treatment | Fluorinated acid | 20.0 | 120 | 70 | 5 | 120 | Inv. |
| 116 | Wet treatment | Fluorinated alcohol | 20.0 | 120 | 70 | 5 | 120 | Inv. |

| | | | | | | | | |
|---|---|---|---|---|---|---|---|---|
| *1: Treating time (sec), *2: Image quality, *3: Cleanliness level, *4: Remarks, Comp.: Comparative example, Inv.: Present invention | | | | | | | | |

As is clear from Table 1, it is to be understood that capsule endoscopes composed of the structure specified in the present invention can wash out a stain with a cleaning solution immediately after photographing, and obtain photographed images exhibiting sharpness in comparison to comparative examples.

## Claims

1. A medical capsule endoscope comprising a surface having a contact angle of not less than 60° and not more than 150° with respect to water.

2. The medical capsule endoscope of Claim 1,
wherein the surface comprises a surface of a lens or an illumination body provided in the medical capsule endoscope.

3. The medical capsule endoscope of Claim 1 or 2,
wherein a chemically bonded fluorine atom is present on the surface.

4. The medical capsule endoscope of any one of Claims 1 - 3,
wherein the surface is treated via a microwave plasma treatment, an electron beam treatment or an X-ray treatment.
